# EUROPEAN PATENT APPLICATION

(11) **EP 2 873 418 A1**
(43) Date of publication of application: **20.05.2015**
(21) Application number: 13193496.0
(22) Date of filing: 19.11.2013
(51) Int. Cl.: A61K 31/4422, A61K 9/00, A61K 47/10, A61P 15/06

(54) **A combination of dosage units for use in the treatment of preterm labour condition**

(71) Applicant: Laboratorio Reig Jofre S.A., 08970 Sant Joan Despí (ES)
(72) Inventor: Navarro Pujol, Francesc, 08970 Sant Joan Despí (ES); Nieto Abad, Carles, 08970 Sant Joan Despí (ES); Alcalde Aguilar, Pilar, 08970 Sant Joan Despí (ES); Gonzalez Plagaro, Jordi, 08970 Sant Joan Despí (ES)
(74) Representative: ZBM Patents - Zea, Barlocci & Markvardsen

(57) **Abstract**

The invention refers to a combination of dosage units of a pharmaceutical or veterinary liquid composition for oral administration for use in the treatment of pre-term labour condition, wherein the treatment comprises administering to a female mammal: (a) First, a dosage unit of the pharmaceutical or veterinary liquid composition comprising 10 mg of nifedipine; followed by (b) From 1 to 4 dosage units of the pharmaceutical or veterinary liquid composition comprising each 7.5 mg of nifedipine, each dosage unit being sequentially administered at an appropriate time period; and wherein the maximum amount of nifedipine administered in a period of an hour is comprised from 17.5 mg to 40 mg.

## Description

The present invention refers to the field of pharmacy. More specifically, the invention relates to the development of a combination of dosage units for the treatment of pre-term labour condition.

### BACKGROUND ART

Pre-term birth, defined as birth at less than 37 weeks of gestation, is the major contributor to perinatal mortality and morbidity, being determinant of adverse infant outcome in terms of both survival and quality of life. Benefits have been identified from prolongation of pregnancy, such as for example by enabling corticosteroids to be administered to hasten fetal lung maturation.

A wide range of active ingredients has been used to inhibit regular uterine contractions causing cervical dilation (pre-term labour) in order to allow time for such co-interventions to occur. Some of the tocolytic agents in current use include betamimetics (such as for example, ritodrine, salbutamol and terbutaline), and calcium channel blockers (such as nifedipine).

Betamimetics have been shown to be effective in delaying delivery by up to seven days when administered intravenously. Nevertheless, they have a high frequency of unpleasant side effects, including severe maternal side effects such as tachycardia, hypotension, tremulousness, a range of biochemical disturbances, and maternal death caused by pulmonary edema.

Alternatively, calcium channel blockers have also been used as tocolytic agents, because of their potent relaxant effect on human myometrium preventing the influx of extracellular calcium ions into the myometrial cell. The most widely used calcium channel blocker is nifedipine which belongs to the dihydropiridine group.

It has been demonstrated that nifedipine is more effective as tocolytic agent than betamimetics agents. Therefore, nifedipine is preferred to other traditional tocolytic agents for the treatment of threatened pre-term labour. The studies reviewed by King et al. used high dosages regimes to stop pre-term contractions ranging from 30 mg/day to 160 mg/day, and dosage regimes of 40-120 mg/day for maintenance treatments lasting for several weeks. (cf. King JF. et al. Cochrane Database of Systematic Reviews 2003, Issue 1. Art. No.: CD002255).

As it was recommended by gynaecologic protocols in case of threat of pre-term birth, the dosage regime of nifedipine to stop pre-term contractions comprises administering doses of from 10 to 30 mg administered orally or sublingually, followed by subsequent doses of from 10 to 20 mg administered orally every four to eight hours for a period of time comprised from 24 to 72 hours.

Nevertheless, the use of short-acting nifedipine in moderate to high doses as those mentioned above still causes some adverse effect associated to its hypotension effect. Therefore, efforts to reduce the adverse effects have been focused on the reduction of the dose of nifedipine. In particular, the last gynaecologic protocols recommend the administration of an initial dose of 10 mg of nifedipine administered orally followed by subsequent orally administered doses of 10 mg each 15-20 minutes if pre-term labour persists, provided that the maximum amount of nifedipine is 40 mg in the first hour.

Clinical and officinal oral liquid compositions of nifedipine have been developed to overcome dosage and administration limitations of solid oral compositions. Liquid oral compositions of nifedipine have showed to be the most biopharmaceutical efficient form of the drug.

The most commonly liquid composition for hospital use with the specific dose required for each individual dose regime is obtained by the preparation of extemporaneous suspensions from bulk nifedipine or immediate release tablets (cf. Helin-Tanninen, M. J. Clin. Pharm. Therap.; 2001, 26, 49-57). These compositions are known to be impaired by the poor dissolution rate of nifedipine which decreases its absorption and impairs the reproducibility of the compositions and thus the variability of its efficacy.

Alternatively, the marketed oral soft gelatine capsules of Adalat^{®} for immediate release of nifedipine have also been used for the preparation of such extemporaneous suspensions. Unfortunately, Adalat comprises such amount of PEG that they cannot be used in cases where a high dose of nifedipine is required. Further, a great variability of nifedipine serum levels have been described in the literature when Adalat compositions are administered, even in the sustained release form (cf. Marin, T. Z. et al, Journal of Obstetrics and Gynaecology, 2007, 27 vol.3, pgs. 260-263). Thus, Adalat compositions do not allow adjusting doses for the requirements of each patient in the treatment and/or prolongation of pre-term labour,

Therefore, even though the advances in the treatment of pre-term labour by the administration of a specific dose regime of nifedipine, it would be still desirable to have a more efficient and safer treatment of pre-term labour.

### SUMMARY OF THE INVENTION

Inventors have found a more efficient and safer combination of dosage units of nifedipine for treating pre-term labour condition in female mammal involving less maternal adverse effects, and reducing the inter-individual variability of the absorption of nifedipine reducing the toxicity risks and inefficacy of the treatment.

The combination of dosage units of the present invention allows administering the same or less amount of active ingredient in the first hour than the treatments of the state of the art. Nevertheless, regardless the nifedipine administered in the first hour, the combination of dosage units of the invention allows reducing the total amount of nifedipine per day. Additionally, the combination of dosage units of the invention allows achieving a lower total drug exposure (AUC) than the treatments currently used (cf. Table 1).Thus, the treatment of the invention involves few or no maternal side effects associated to active ingredient.

The combination of dosage units of the invention also allows achieving a higher maximum concentration of nifedipine (Cₘₐₓ) than the known treatments, becoming more efficient in postponing and/or stopping the pre-term labour (cf. Table 1).

Further, the combination of the present invention which comprises administering first, a dosage unit comprising 10 mg of nifedipine; followed by four dosage units comprising each 7.5 mg of nifedipine, allows administering an additional dose, maintaining the maximum amount of nifedipine administered in the first hour (i.e. 40 mg). It is advantageous because there is an extra opportunity of success, without increasing the adverse effects associated to nifedipine, in fact, the adverse effects can be reduced.

Thus, the invention provides a combination of dosage units of a pharmaceutical or veterinary liquid composition for oral administration comprising a) nifedipine in an amount comprised from 0.1% to 1% w/w; b) ethanol in an amount comprised from 38% to 58% w/w; c) water in an amount comprised from 4% to 12% w/w; d) glycerine in an amount comprised from 30% to 50% w/w; and e) optionally, other pharmaceutically or veterinary acceptable excipients or carriers; being the sum total of components 100% w/w, for use in the treatment of pre-term labour condition, wherein the treatment comprises administering to a female mammal: (a) First, a dosage unit of the pharmaceutical or veterinary liquid composition comprising 10 mg of nifedipine; followed by (b) From 1 to 4 dosage units of the pharmaceutical or veterinary liquid composition comprising each 7.5 mg of nifedipine, each dosage unit being sequentially administered at an appropriate time period; and wherein the maximum amount of nifedipine administered in a period of an hour is comprised from 17.5 mg to 40 mg.

Preferably, the dosage units of the invention are substantially free of polyethylenglycol which means that the amount of polyethylene glycol is comprised from 0% to 0.1% w/w.

Also preferably, the dosage units of the invention are substantially free of a derivate of polyethylene glycol selected from the group consisting of polyethylene glycol alkyl ethers, polyethylene glycol fatty acid esters, polyethylene glycol sorbitan fatty acid esters, and polyethylene glycol oxystearates which mean that the amount of the derivatives of polyethylene glycol is comprised from 0% to 0.1 % w/w;

### BRIEF DESCRIPTION OF THE DRAWINGS

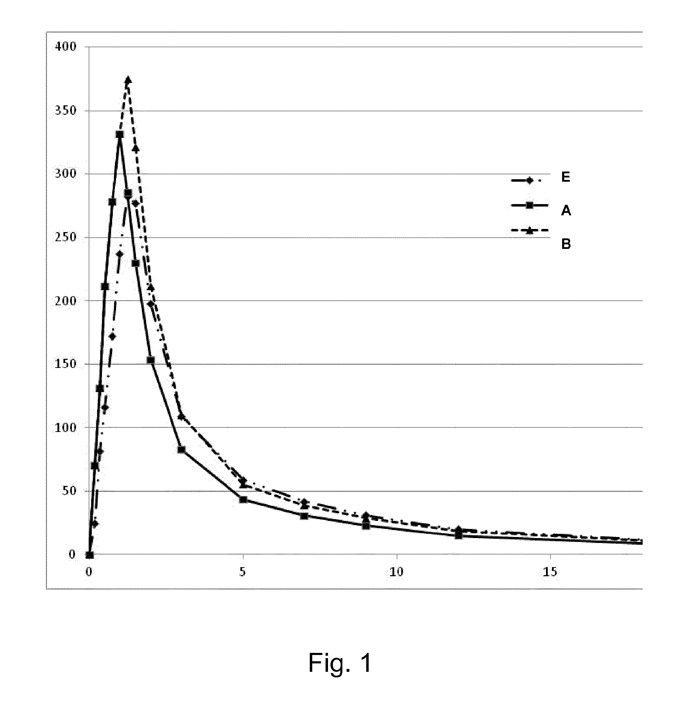

Fig. 1 represents the plasma concentration-time curves after oral administration of the combination of dosage units corresponding to treatment A of the invention (■ symbol), treatment B of the invention (▲ symbol), and comparative treatment E (0 symbol).

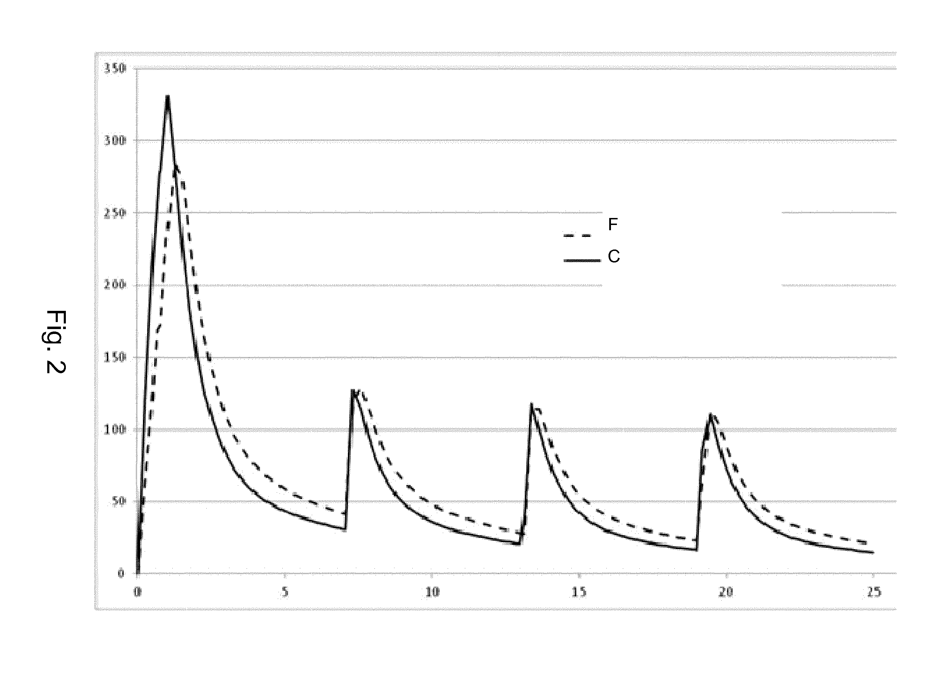

Fig. 2 represents the plasma concentration-time curves after oral administration of the combination of dosage units corresponding to treatment C of the invention (continuous line), and Comparative treatment F (discontinuous line).

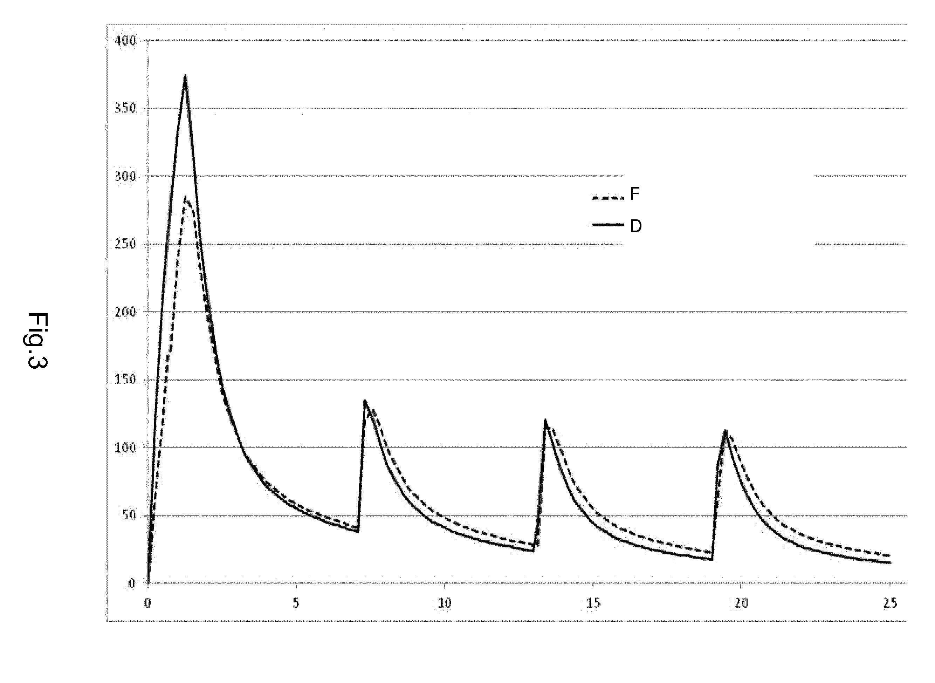

Fig. 3 represents the plasma concentration-time curves after oral administration of the combination of dosage units corresponding to treatment D of the invention (continuous line), and Comparative treatment F (discontinuous line).

### DETAILED DESCRIPTION OF THE INVENTION

In the present invention, the term "treating" the pre-term labour refers to the fact of delaying the pre-term birth by postponing or alternatively by stopping the pre-term labour of a female mammal.

The term "pharmaceutical dosage unit" refers to a pharmaceutical entity that comprises a pharmacologically active ingredient and pharmaceutical acceptable excipients or carriers and which is actually to be administered to, or to be taken by, a human. The term "pharmaceutical dosage unit" also encompasses non-reusable packaging as well, especially when pharmaceutical composition is individually packaged. Further, the "pharmaceutical dosage unit" of the invention can be obtained from a larger dosage unit prepared in advanced. These larger dosage units are known as "multidose dosage units".

The term "veterinary dosage unit" refers to a veterinary entity that is comprised of a pharmacologically active ingredient and which is actually to be administered to, or to be taken by, an animal. The term "veterinary dosage unit" also encompasses non-reusable packaging as well, especially when veterinary composition is individually packaged. Further, the "veterinary dosage unit" of the invention can be obtained from a larger dosage unit prepared in advanced. These larger dosage units are known as "multidose units".

The expression "pharmaceutically or veterinary acceptable excipients or carriers" refers to pharmaceutically or veterinary acceptable materials, compositions or vehicles. Each component must be pharmaceutically or veterinary acceptable in the sense of being compatible with the other ingredients of the pharmaceutical or veterinary dosage unit. It must also be suitable for use in contact with the tissue or organ of humans and animals without excessive toxicity, irritation, allergic response, immunogenicity or other problems or complications commensurate with a reasonable benefit/risk ratio.

In an embodiment the mammal is a human, and the composition of the invention is a pharmaceutical liquid composition for oral administration

In an embodiment of the invention, the treatment comprises postponing the pre-term labour condition; preferably the treatment comprises postponing the pre-term labour condition from 24 to 48 hours. In an alternative embodiment the treatment comprises stopping the pre-term labour condition. It is advantageous because the pre-term birth can be delayed long enough for the necessary co-interventions are carried out or even stopped until the birth can be considered a full term birth, which is until 37 weeks of gestation.

In an embodiment, in the treatment of the invention each of the subsequent dosage units (b) comprising 7.5 mg of nifedipine is sequentially administered in a period of time comprised from 5 to 20 min; preferably administered in a period of time comprised from 10 to 18 min; more preferably each 15 min, being the maximum amount of nifedipine administered in a period of an hour comprised from 17.5 mg to 40 mg.

In a particular embodiment, the treatment of the invention comprises administering (a) first, a dosage unit comprising 10 mg of nifedipine; followed by (b) one dosage unit comprising 7.5 mg of nifedipine. In another particular embodiment, the treatment of the invention comprises administering (a) first, a dosage unit comprising 10 mg of nifedipine; followed by (b) two dosage units comprising each 7.5 mg of nifedipine. In another particular embodiment, the treatment of the invention comprises administering (a) first, a dosage unit comprising 10 mg of nifedipine; followed by (b) three dosage units comprising each 7.5 mg of nifedipine. In another particular embodiment, the treatment of the invention comprises administering (a) first, a dosage unit comprising 10 mg of nifedipine; followed by (b) four dosage units comprising each 7.5 mg of nifedipine.

In an embodiment of the invention, the treatment of the invention further comprises administering dosage units of maintenance. Thus, the combination of dosage units for use in the treatment of pre-term labour conditions further comprises administering one or more additional dosage units (c) at an appropriate time period, being the maximum amount of nifedipine to be administered to a female mammal in a period of a day of 160 mg per day; wherein each additional dosage unit (c) administered comprises an amount of nifedipine from 7.5 mg to 22.5 mg of nifedipine; preferably each additional dosage unit (c) administered comprises an amount of nifedipine from 7.5 mg to 15 mg of nifedipine; more preferably each additional dosage unit (c) administered comprises an amount of nifedipine of 7.5 mg.

In another embodiment, in the combination of dosage units for use in the treatment of pre-term labour conditions which further comprises administering one or more additional dosage units (c), the appropriate time period is comprised from 6 to 8 hours.

In another embodiment, in the combination of dosage units for use in the treatment of pre-term labour conditions which further comprises administering one or more additional dosage units (c), the treatment is carried out for a period of 72 hours; preferable for a period of 48 hours.

In another embodiment, in the combination of dosage units for use in the treatment of pre-term labour conditions which further comprises administering one or more additional dosage units (c), being the maximum amount of nifedipine to be administered to a female mammal in a period of a day of 100 mg per day; preferably of 70mg per day.

In another embodiment, in the combination of dosage units for use in the treatment of pre-term labour conditions which further comprises administering one or more additional dosage units (c), each additional dosage unit (c) comprises an amount of nifedipine of 7.5 mg; and is administered in a time period from 6 to 8 hours; and the treatment is carried out in a period of 72 hours.

In another embodiment, in the combination of dosage units for use in the treatment of pre-term labour conditions which further comprises administering one or more additional dosage units (c), each additional dosage unit (c) comprises an amount of nifedipine of 7.5 mg; and is administered each 6 hours; and the treatment is carried out in a period of 72 hours.

In the present invention, unless explicitly stated, all percentages are given in weight with regard to the total weight of the unitary liquid pharmaceutical or veterinary dosage unit.

In the present invention, the term "liquid" refers to a pharmaceutical or veterinary composition for oral administration intended for oral use wherein the pharmaceutical or veterinary product, i.e. nifedipine, and the excipients are partially or totally dissolved in a solvent system; preferably totally dissolved in a solvent system. In a preferred embodiment, the pharmaceutical or veterinary liquid composition for oral administration is a solution.

In a particular embodiment of the invention, the dosage units of the invention is substantially free of any other solubilizing agent or diluent that is that the amount of any other solubilizing agent or diluent is comprised from 0% to 0.1 % w/w.

The amount of glycerine in each dosage unit of the invention is comprised from 30% to 50%; preferably, from 40% to 46%; more preferably from 42% to 44%.

The amount of ethanol in each dosage unit of the invention is comprised from 38% to 58%; preferably, from 43% to 53%; more preferably, from 45% to 50%.

The amount of water in each dosage unit of the invention is comprised from 4% to 12%; preferably, from 6% to 10%; more preferably, from 7% to 9%.

In a particular embodiment, each dosage unit of the present invention has the following composition: a) nifedipine in an amount comprised from 0.1% to 1.0%; b) ethanol in an amount comprised from 43% to 53%; c) water in an amount comprised from 6% to 10%; and d) glycerine in an amount comprised from 40% to 46%.

In another particular embodiment, each dosage unit of the present invention is a solution having the following composition: a) nifedipine in an amount comprised from 0.3% to 0.7%; b) ethanol in an amount comprised from 45% to 50%; c) water in an amount comprised from 7% to 9%; d) glycerine in an amount comprised from 42% to 44%. Preferably, each dosage unit further comprises e) at least one preservative in an amount comprised from 0.05% to 0.5%; f) at least one sweetener in an amount comprised from 0.02% to 0.8%; and g) optionally at least one colouring or flavouring agents, each of them in an amount comprised from 0.01 % to 0.07%.

In an embodiment of the invention, each dosage unit of the present invention can also comprise pharmaceutically or veterinary acceptable excipients or carriers such as preservatives, sweeteners, colouring and flavouring agents. Suitable preservatives for each dosage unit of the invention can be selected from phenoxyethanol, imidurea, methylparaben, ehylparaben, propylparaben, butylparaben and its salts, and mixtures thereof. Preferred preservatives are ethyl and methylparaben salts and mixtures thereof.

Suitable sweeteners for each dosage unit of the invention can be, for example, maltitol, aspartame, erythritol, lactitol, fructose, sucrose, alitame, hesperedine, neohesperedin dihydrochalcone, cyclamate salts, saccharin or acesulfame.

Further, each dosage unit of the invention can comprise flavouring and colouring agents selected from those known by the person skilled in the art, in order to improve the taste and appearance of the product.

Other conventional excipients may be employed in the dosage units of the present invention, for example, buffering systems, viscosity enhancing agents, additional sweeteners, and their mixtures.

In all the previous embodiments, the sum total of components is 100% w/w,

Dosage units of the present invention can be prepared according to methods well known in the state of the art. The appropriate excipients and/or carriers, and their amounts, can readily be determined by those skilled in the art.

As an example, a dosage unit according to the invention can be prepared by mixing an ethanol aqueous solution comprising an amount of ethanol comprised from 43% to 53% of the total composition, which may comprise sweeteners, preservatives or flavouring agents, first with nifedipine in an amount comprised from 0.1 % to 1% of the total composition, and optionally, then adding glycerine comprised from 40% to 46% of the total composition. Afterwards, water is added until a total content comprised from 6% to 10% of the total composition, optionally with additional sweeteners or flavouring agents dissolved. The sum total of components is 100%

The liquid solution can be packaged in unitary dose or multidose containers, for example, ampoules, vials, prefilled syringes, cartridges or bags. The final solution may be dosed and administrated by a syringe, dropper, or any suitable quantitative device. Preferably, the solution is dosed by a syringe. The present invention could be also formulated as the use of nifedipine for the preparation of a medicament for the treatment of the pre-term labour condition, wherein the medicament is a pharmaceutical or veterinary liquid composition for oral administration comprising a) nifedipine in an amount comprised from 0.1 % to 1% w/w; b) ethanol in an amount comprised from 38% to 58% w/w; c) water in an amount comprised from 4% to 12% w/w; d) glycerine in an amount comprised from 30% to 50% w/w; and e) optionally, other pharmaceutically or veterinary acceptable excipients or carriers; being the sum total of components 100% w/w; and wherein the treatment comprises administering to a female mammal: (a) First, a dosage unit of the pharmaceutical or veterinary liquid composition comprising 10 mg of nifedipine; followed by (b) from 1 to 4 dosage units of the pharmaceutical or veterinary liquid composition comprising each 7.5 mg of nifedipine, each dosage unit being sequentially administered at an appropriate time period; being the maximum amount of nifedipine administered in a period of an hour comprised from 17.5 mg to 40 mg.

It also relates to a method for the treatment of a female mammal suffering from a pre-term labour condition, wherein the method comprises administering to said mammal: (a) first, a dosage unit of a pharmaceutical or veterinary liquid composition comprising 10 mg of nifedipine; followed by (b) from 1 to 4 dosage units of a pharmaceutical or veterinary liquid composition comprising each 7.5 mg of nifedipine, each dosage unit being sequentially administered at an appropriate time period; being the maximum amount of nifedipine administered in a period of an hour comprised from 17.5 mg to 40 mg, and wherein the pharmaceutical or veterinary liquid composition is for oral administration comprising a) nifedipine in an amount comprised from 0.1% to 1% w/w; b) ethanol in an amount comprised from 38% to 58% w/w; c) water in an amount comprised from 4% to 12% w/w; d) glycerine in an amount comprised from 30% to 50% w/w; and e) optionally, other pharmaceutically or veterinary acceptable excipients or carriers; being the sum total of components 100% w/w.

Throughout the description and claims the word "comprise" and variations of the word, are not intended to exclude other technical features, additives, components, or steps. Furthermore, the word "comprise" encompasses the case of "consisting of'. Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### EXAMPLES

### EXAMPLE 1. Oral solution of nifedipine

### 1.1. PROCESS FOR THE PREPARATION

Each dosage units (a), (b), and (c) can be prepared from the components of the following table:

**"q.s." means "as needed".**

| **Ingredients** | **Amount (g)** |
|---|---|
| Nifedipine | 0.150 |
| Ethanol 96° | 14.000 |
| Glycerine | 12.600 |
| Sodium cyclamate | 0.150 |
| Sodium saccharine | 0.015 |
| Ethylparaben | 0.0525 |
| Colouring agent E-110 | 0.006 |
| Flavouring Agent | 0.02 |
| Purified water | q.s. 30.0 ml (about 2.4 g) |

The process for the preparation of each dosage units (a), (b), or (c) was as follows:

Sodium cyclamate and the colouring agent E-110 were dissolved in water to obtain the aqueous solution I.

An alcoholic solution was prepared dissolving ethylparaben, sodium saccharine and the flavouring agent in ethanol 96° to obtain the alcoholic Solution I.

Nifedipine were added to the alcoholic Solution I and the mixture was homogenized. Then, glycerine was added and the solution was mixed to obtain the alcohol Solution II. When the mixture became homogeneous, the aqueous solution I was added to the alcoholic solution II and the mixture was homogenized. Volume was corrected with water and the solution was mixed until obtaining a suitable homogeneous solution (solution A).

From the resulting homogeneous solution A, a fraction of 2 ml was extracted to obtain the dosage unit (a) comprising 10 mg of nifedipine of the present invention.

Further, from the resulting homogeneous solution A, a fraction of 1.5 ml was extracted to obtain the dosage unit (b) comprising 7.5 mg of nifedipine of the present invention.

For the additional dosage units (c) to be used according to the invention the corresponding fraction is extracted to obtain the dosage unit comprising the desired amount of nifedipine.

### EXAMPLE 2. Comparative bioavailability and pharmacokinetic Assay

### A. METHOD

Bioavailability means the rate and extent to which the active ingredient is absorbed from a pharmaceutical form and becomes available at the systemic circulation. Bioavailability is assessed by serial measurements of the drug in the systemic circulation. These serial measurements provide a plasma concentration-time curve from which some important pharmacokinetic parameters can be calculated, including dose-corrected area under curve (AUC), the maximum observed concentration (Cₘₐₓ) and the time when Cₘₐₓ is reached (tₘₐₓ). The AUC provides an estimate of the amount of drug absorbed in the systemic circulation while tₘₐₓ and Cₘₐₓ reflects the rate of absorption.

All pharmacokinetic parameters were determined using WinNonlin-Pro 2.1 software.

The aim of the assay was to test the tocolytic efficacy and the side effects associated to the tested dose regimen.

### B. TREATMENT

The following treatments were assayed:

### Treatment A

The dosage unit (a) comprising 10 mg of nifedipine and the dosage unit (b) comprising 7.5 mg of nifedipine obtained both in Example 1 are administered with the following dose regime:
- (i): 1 dosage unit (a) of 10 mg administered at 0 hour; and
- (ii): 3 dosage units (b) of 7.5 mg administered at 0.25 hour, 0.50 hour, and 0.75 hour; and

### Treatment B

The dosage unit (a) comprising 10 mg of nifedipine and the dosage unit (b) comprising 7.5 mg of nifedipine obtained both in Example 1 are administered with the following dose regime:
- (i): 1 dosage unit (a) of 10 mg administered at 0 hour; and
- (ii): 4 dosage units (b) of 7.5 mg administered at 0.25 hour, 0.50 hour, and 0.75 hour; and 1 hour; and

### Treatment C

The dosage unit (a) comprising 10 mg of nifedipine, the dosage unit (b), and dosage unit (c) comprising 7.5 mg of nifedipine obtained in Example 1 are administered with the following dose regime:
- (i): 1 dosage unit (a) of 10 mg administered at 0 hour;
- (ii): 3 dosage units (b) of 7.5 mg administered at 0.25 hour, 0.50 hour, and 0.75 hour; and
- (iii): 3 dosage units (c) of 7.5 mg administered each 6 hours (that is, at 7 hour, 13 hour, and 19 hour).

### Treatment D

The dosage unit (a) comprising 10 mg of nifedipine, the dosage unit (b), and the dosage unit (c) comprising 7.5 mg of nifedipine obtained in Example 1 are administered with the following dose regime:
- (i): 1 dosage unit (a) of 10 mg administered at 0 hour;
- (ii): 4 dosage units (b) of 7.5 mg administered at 0.25 hour, 0.50 hour, 0.75 hour; and 1 hour; and
- (iii): 3 dosage units (c) of 7.5 mg administered each 6 hours (that is, at 7 hour, 13 hour, and 19 hour).

### Comparative Treatment E

A soft-gel capsule of Adalat containing 10 mg is administered with the following dose regime:
- (i): 4 doses of 10 mg administered at 0 hour; 0.33 hour, 0.67 hour, and 1 hour.

### Comparative Treatment F

A soft-gel capsule of Adalat containing 10 mg of nifedipine is administered with the following dose regime:
- (i): 4 doses of 10 mg administered at 0 hour; 0.33 hour, 0.67 hour, and 1 hour;
- (ii): 3 doses of 10 mg administered each 6 hours (that is, at 7 hour, 13 hour, and 19 hour).

### C. RESULTS

The amount of nifedipine administered in the first hour, the total amount of nifedipine administered per day, as well as the values of Cₘₐₓ and AUC are summarizes in Table 1:

**Table 1**

| Treatment | Amount of nifedipine | | Pharmacokinetic parameters | |
|---|---|---|---|---|
| | Total amount in the first hour (mg) | Total amount per day (mg) | Cmax (ng/ml) | AUC₀ (ng.h/ml) |
| A^{(a)} | 32.5 | - | 331.561 | |
| B^{(a)} | 40 | - | 374.299 | |
| C | 32.5 | 55 | 331.561 | 1465.96 |
| D | 40 | 62.5 | 374.299 | |
| Comparative E^{(a)} | 40 | - | 284.487 | |
| Comparative F | 40 | 70 | 284.487 | 1676.40 |

| | | | | |
|---|---|---|---|---|
| (a) The treatment does not comprise additional administration of nifedipine after the first hour. | | | | |

As extracted from the result data in Table 1, treatment of the invention (C) shows a lower AUC (i.e. 1465.96) than comparative treatment (F) (i.e. 1676.40). Additionally, Even when the treatment of the invention comprises the administration of 4 doses of nifedipine in the first hour (treatment D), the total amount of nifedipine administered per day in the treatments of the invention (C-D) (55 and 62.5 mg) is lower than the total amount in the comparative treatment (70 mg); and even less than or about 60 mg/day. As a consequence, treatments of the invention involve few or no maternal side effects associated to the therapeutic activity of the active ingredient.

On the other hand, treatments of the invention (A-D) show an increased Cₘₐₓ (i.e. 331.561, and 374.299) than comparative treatments (E-F) (i.e. 284.487). As a consequence, treatments of the invention are more efficient than the ones disclosed in the state of the art for use in the treatment for pre-term labour.

The results show that the dosage regime of the present invention for use in the treatment of the pre-term labour condition is more efficient and safer than the ones of the state of the art.

## Claims

1. A combination of dosage units of a pharmaceutical or veterinary liquid composition for oral administration comprising
a) nifedipine in an amount comprised from 0.1% to 1% w/w;
b) ethanol in an amount comprised from 38% to 58% w/w;
c) water in an amount comprised from 4% to 12% w/w;
d) glycerine in an amount comprised from 30% to 50% w/w; and
e) optionally, other pharmaceutically or veterinary acceptable excipients or carriers;
being the sum total of components 100% w/w,
for use in the treatment of pre-term labour condition,
wherein the treatment comprises administering to a female mammal:
(a) First, a dosage unit of the pharmaceutical or veterinary liquid composition comprising 10 mg of nifedipine; followed by
(b) From 1 to 4 dosage units of the pharmaceutical or veterinary liquid composition comprising each 7.5 mg of nifedipine, each dosage unit being sequentially administered at an appropriate time period; and
wherein the maximum amount of nifedipine administered in a period of an hour is comprised from 17.5 mg to 40 mg.

2. The combination of dosage units for use according to claim 1, wherein the mammal is a human.

3. The combination of dosage units for use according to any of the claims 1-2, wherein each dosage unit (b) is sequentially administered at a time period comprised from 5 to 20 min.

4. The combination of dosage units for use according to claim 3, wherein each dosage unit (b) is sequentially administered at a time period comprised from 10 to 18 min.

5. The combination of dosage units for use according to any of the claims 1-4, wherein the treatment comprises administering:
(a) First, a dosage unit comprising 10 mg of nifedipine; followed by (b) one dosage unit comprising 7.5 mg of nifedipine; or alternatively
(a) First, a dosage unit comprising 10 mg of nifedipine; followed by (b) two dosage units comprising each 7.5 mg of nifedipine.

6. The combination of dosage units for use according to any of the claims 1-4, wherein the treatment comprises administering: (a) First, a dosage unit comprising 10 mg of nifedipine; followed by (b) three dosage units comprising each 7.5 mg of nifedipine.

7. The combination of dosage units for use according to any of the claims 1-4, wherein the treatment comprises administering: (a) First, a dosage unit comprising 10 mg of nifedipine; followed by (b) four dosage units comprising each 7.5 mg of nifedipine.

8. The combination of dosage units for use according any of the claims 1-7, further comprises administering one or more additional dosage units (c) at an appropriate time period, being the maximum amount of nifedipine to be administered to a female mammal in a period of a day of 160 mg per day; wherein each additional dosage unit (c) administered comprises an amount of nifedipine from 7.5 mg to 22.5 mg of nifedipine.

9. The combination of dosage units for use according to claim 8, wherein the appropriate time period is comprised from 6 to 8 hours.

10. The combination of dosage units for use according to any of the claims 8-9, wherein the treatment is carried out for a period of 72 hours.

11. The combination of dosage units for use according to any of the claims 1-10, wherein the amount of glycerine in the pharmaceutical or veterinary liquid composition is comprised from 40% to 46% w/w.

12. The combination of dosage units for use according to any of the claims 1-11, wherein the amount of ethanol in the pharmaceutical or veterinary liquid composition is comprised from 45% to 53% w/w.

13. The combination of dosage units for use according to any of the claims 1-12, wherein the pharmaceutical or veterinary liquid composition comprises:
a) nifedipine in an amount comprised from 0.3% to 0.7% w/w;
b) ethanol in an amount comprised from 45% to 50% w/w;
c) water in an amount comprised from 7% to 9% w/w; and
d) glycerine in an amount comprised from 42% to 44% w/w.

14. The combination of dosage units for use according to any of the claims 1-13, wherein the treatment comprises postponing the pre-term labour condition from 24 to 48 hours.

15. The combination of dosage units for use according to any of the claims 1-13, wherein the treatment comprises stopping the pre-term labour condition.
